(19) Europäisches Patentamt
European Patent Office
Office européen des brevets

(11) Numéro de publication: **0 112 262**
**A1**

(12) **DEMANDE DE BREVET EUROPEEN**

(21) Numéro de dépôt: 83420185.7

(22) Date de dépôt: 08.12.83

(51) Int. Cl.³: C 07 D 213/82
C 07 D 401/06, A 01 N 43/40
A 01 N 43/64

(30) Priorité: 13.12.82 FR 8221078

(43) Date de publication de la demande: 27.06.84 Bulletin 84/26

(84) Etats contractants désignés: AT BE CH DE FR GB IT LI LU NL SE

(71) Demandeur: RHONE-POULENC AGROCHIMIE
14-20, rue Pierre Baizet
F-69009 Lyon(FR)

(72) Inventeur: De Reinach Hirtzbach, François
5 Quai Général Sarrail
F-69006 Lyon(FR)

(72) Inventeur: Ambrosi, Dominique
15 Allée des Hautinières
F-69260 Charbonnières les Bains(FR)

(74) Mandataire: Chaumette, Michel et al,
RHONE-POULENC AGROCHIMIE BP 9163-09
F-69263 Lyon Cedex 1(FR)

(54) **Nouveaux dérivés de la benzylcarbamoylpyridine.**

(57) Ces nouveaux dérivés répondent à le formule générale (I)

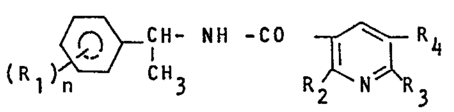

(I)

$R_1$ : halogène, alkyle ($C_1$-$C_6$) éventuellement halogéné ; alkoxy ($C_1$-$C_4$)

$R_2$ et $R_3$ : alkyle ($C_1$-$C_3$), alkoxy ($C_1$-$C_4$), alkoxyalkyle ($C_2$-$C_8$)

$R_4$ : acyle, azidométhyle, alkoxycarbonylméthyl ($C_3$-$C_8$), hydroxyalkyle ($C_2$-$C_5$), halogénoalkyle ($C_2$-$C_5$), alkyle ($C_1$-$C_4$) éventuellement substitué par un radical hétérocyclique, alcynyloxyalkyle $C_4$-$C_8$

n : 0 à 5

Ils sont utilisables pour le désherbage sélectif de cultures telles que coton, et soja.

EP 0 112 262 A1

Croydon Printing Company Ltd.

La présente demande de brevet concerne de nouveaux dérivés de la benzylcarbamoylpyridine, ainsi que leurs procédés de préparation, les compositions herbicides les contenant et leur application pour le désherbage sélectif de cultures, notamment le coton, et le soja.

Ces nouveaux dérivés répondent à la formule générale (I)

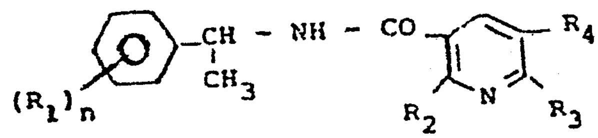

(I)

dans laquelle :

$R_1$ représente un atome d'halogène ou un radical alkyle comportant de 1 à 6 atomes de carbone, éventuellement substitué par un ou plusieurs atomes d'halogène (par exemple le radical trifluorométhyle), ou un radical alkoxy comportant de 1 à 4 atomes de carbone,

$R_2$ et $R_3$, identiques ou différents représentent le radical alkyle comportant de 1 à 3 atomes de carbone, ou un radical alkoxy comportant de 1 à 4 atomes de carbone, ou alkoxyalkyle comportant de 2 à 8 atomes de carbone,

$R_4$ représente un radical choisi parmi les radicaux :

- acyle (de préférence alcanoyle comportant de 1 à 6 atomes de carbone ou benzoyle éventuellement substitué, par exemple par un ou plusieurs atomes d'halogène)
- azidométhyle,
- alkoxycarbonylméthyle comportant de 3 à 8 atomes de carbone,
- hydroxyalkyle comportant de 2 à 5 atomes de carbone,
- halogénoalkyle comportant de 2 à 5 atomes de carbone,
- alkyle comportant de 1 à 4 atomes de carbone,

- méthyle substitué par un radical hétérocyclique, comportant de 5 à 6 chainons et de 1 à 3 hétéroatomes choisis parmi les atomes d'oxygène, de soufre et d'azote,
- vinyle,
- et les radicaux alcynyloxyalkyle comportant de 4 à 9 atomes de carbone.

n est un nombre entier de 0 à 5, valeurs limites comprises, étant entendu que, lorsque plusieurs substituants $R_1$ sont présents, ces substituants peuvent être soit identiques soit différents.

Les composés répondant à la formule (I) peuvent former des sels avec des acides appropriés, qui peuvent être soit des acides minéraux, comme par exemple l'acide chlorhydrique, l'acide sulfurique, l'acide phosphorique, soit des acides organiques comme par exemple l'acide succinique, l'acide fumarique, l'acide oxalique, l'acide benzoïque, l'acide tartrique. Ces divers sels sont également inclus dans le cadre de la présente invention, tout particulièrement lorsqu'ils sont acceptables en agriculture.

La demande de brevet allemand DE-B-1 116 669 décrit la préparation d'(alpha-alkylbenzyl)carbamoyl-3 pyridines utilisables comme médicaments, par réaction de l'acide nicotinique (ou acide pyridine-3 carboxylique), avec la benzylamine de formule :

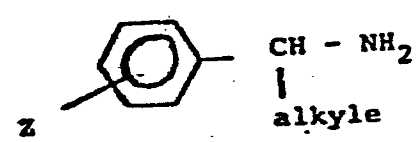

dans laquelle Z représente un atome d'hydrogène ou d'halogène ou un radical alkyle ou alkoxy inférieur et "alkyle" représente un radical alkyle contenant au moins deux atomes de carbone.

La demande de brevet européen EP-A-0 044 262 décrit, en tant qu'herbicides divers dérivés de l'aniline, parmi lesquels plusieurs dérivés de la N-(phényl)carbamoyl-3 diméthyl-2,6 pyridine.

Les composés selon la présente invention sont différents de ceux décrits dans ces deux demandes de brevet. Leur activité herbicide est très sensiblement supérieure à celle des N-(phényl)carbamoyl-3 diméthyl-2,6 pyridines homologues décrites dans la demande de brevet européen n° 0 044 262.

Parmi les composés selon la formule (I), une sous-famille préférée du fait de ses propriétés herbicides est constituée par les composés pour lesquels :

- n est égal à 0 ou 1 ou 2 ou 3
- $R_1$ représente un atome d'halogène ou un radical méthyle
- $R_2$ représente le radical méthyle,
- $R_3$ représente un radical méthyle ou méthoxyméthyle
- $R_4$ représente un radical alcanoyle comportant de 1 à 6 atomes de carbone, azidométhyle, halogénoalkyle comportant de 2 à 5 atomes de carbone, ou alkyle comportant de 1 à 3 atomes de carbone,
- et par les sels acceptables en agriculture de ces composés.

Parmi ces composés préférés, une sous-famille préférée du fait de ses excellentes propriétés herbicides est constituée par les composés selon la formule (I) pour lesquels :

- n est égal à 0,
- $R_2$ et $R_3$ représentent le radical méthyle,
- $R_4$ représente un radical alcanoyle comportant de 1 à 3 atomes de carbone, azidométhyle, halogénoalkyle comportant de 2 à 4 atomes de carbone, ou alkyle comportant de un à trois atomes de carbone ;
- et les sels acceptables en agriculture de ces composés.

Les composés de formule (I) présentent en position alpha du noyau phényle un atome de carbone substitué de façon asymétrique et peuvent donc se présenter sous différentes formes racémiques ou sous forme d'antipodes optiques. Ces différentes formes isomères, optiquement actives ou racémiques, ainsi que leurs sels, obtenus comme indiqués plus haut, sont compris dans le cadre de l'invention.

Parmi ces différentes formes, les isomères optiques présentant la même configuration optique que la (S)-alpha-méthylbenzylamine sont généralement préférés pour leurs propriétés herbicides.

Les composés selon la formule (I) peuvent être préparés selon l'un ou l'autre des procédés a-i décrits ci-après :

Dans ce qui suit, pour plus de commodité, la lettre R représentera le radical de formule (II)

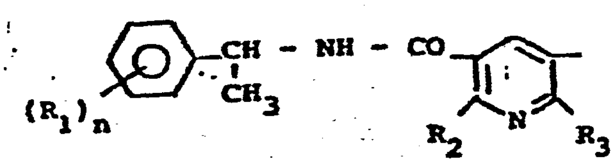 (II)

$R_1$, $R_2$, $R_3$ et n ayant la même signification que dans la formule (I).

Procédé a

Les composés selon la formule (I), pour lesquels $R_4$ représente un radical acyle et qui répondent donc à la formule (III) :

$$R\text{-}CO\text{-}R_5 \qquad (III)$$

dans laquelle $R_5$ représente un reste organique (de préférence un radical alkyle ou phényle éventuellement substitué) et R a la même signification que dans la formule (II), peuvent être obtenus par action du composé organomagnésien de formule (IV) :

$R_5$ MgX (IV)

dans laquelle $R_5$ a la même signification que dans la formule (III) et X représente un atome d'halogène (de préférence le brome ou l'iode) sur l'alkoxycarbonylpyridine de formule (V) :

$R\text{-}COO\text{-}R_6$ (V)

dans laquelle R a la même signification que précédemment et $R_6$ représente un radical alkyle comportant de 1 à 6 atomes de carbone (de préférence méthyle ou éthyle), puis par hydrolyse de l'alcoolate halogénomagnésien intermédiairement formé.

La réaction de (IV) avec (V) s'effectue avantageusement en milieu anhydre dans un éther tel que l'éther diéthylique ou isopropylique, ou dans le tétrahydrofuranne, ou dans un mélange des ces éthers.

L'hydrolyse de l'alcoolate halogénomagnésien s'effectue avantageusement par addition au mélange réactionnel d'une solution aqueuse de chlorure d'ammonium ou d'un acide minéral dilué tel que l'acide chlorhydrique ou l'acide sulfurique.

L'alkoxycarbonylpyridine (V) peut être obtenue par déshydrogénation de la benzylcarbamoyl-3 alkoxycarbonyl-5 dihydro-1,4 pyridine de formule (VI) :

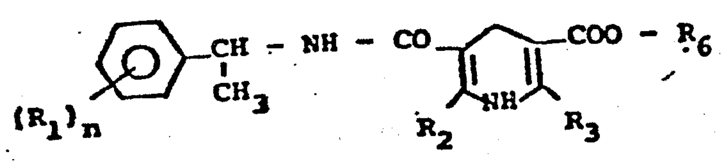

(VI)

dans laquelle $R_1$, $R_2$, $R_3$ et n ont la même signification que dans la formule (I) et $R_6$ la même signification que dans la formule (V).

Cette déshydrogénation peut être effectuée par action d'un oxydant tel que $KMnO_4$ sur le composé (VI). Ce composé (VI) est lui même obtenu selon une méthode analogue à celle décrite dans la demande de brevet européen n°81420106.7 pour la préparation de la dihydro-1,4 N-(diéthyl-2,6 phényl) carbamoyl-3 éthoxycarbonyl-5 lutidine-2,6.

Procédé b

Les composés selon la formule (I) pour lesquels $R_4$ représente un radical azidométhyle et qui répondent donc à la formule (VII) :

$$R\text{-}CH_2\text{-}N_3 \qquad (VII)$$

dans laquelle R a la même signification que dans la formule (II), peuvent être obtenus par action d'un azidure de métal alcalin, tel que l'azidure de sodium $NaN_3$, sur la chlorométhylpyridine de formule (VIII) :

$$R\text{-}CH_2\text{-}Cl \qquad (VIII)$$

dans laquelle R a la même signification que précédemment.

La réaction s'effectue avantageusement en milieu solvant organique inerte,
à une température de l'ordre de 10 à 40°C.

Elle s'effectue de préférence dans un mélange solvant susceptible de dissoudre les deux réactifs, par exemple les mélanges eau et alcanols inférieurs, le mélange DMSO et eau, dès la température ordinaire.
La chlorométhylpyridine (VIII) peut être obtenue à partir de l'ester de formule (XVII) :

$$R\text{-}COO\text{-}R_6 \qquad (XVII)$$

dans laquelle R a la même signification que précédemment et $R_6$ a la même signification que dans la formule (V), par

réduction de cet ester en alcool de formule :

$$R-CH_2-OH$$

et enfin chloration de cet alcool pour donner la chlorométhylpyridine (VIII).

L'ester de formule (XVII) peut être préparé selon la méthode décrite dans le procédé a) décrit plus haut.

Procédé c

Les composés selon la formule (I) pour lesquels $R_4$ représente un radical méthyle substitué par un radical hétérocyclique (par exemple le radical triazolyle) et qui répondent à la formule :

$$R-CH_2-A$$

dans laquelle R a la même signification que dans la formule (II) et A représente un radical hétérocyclique comportant de 5 à 6 chainons et de 1 à 3 hétéroatomes choisis parmi les atomes d'oxygène, soufre et azote, peuvent être obtenus par réaction de la chlorométhylpyridine de formule (VIII) dans laquelle R a la même signification que précédemment, sur le composé hétérocyclique de formule AH dans laquelle A a la même signification que précédemment.

La réaction s'effectue avantageusement dans un solvant aprotique polaire tel que le diméthylformamide, le diméthylsulfoxyde, le diméthylacétamide, en présence d'un agent anionisant de basicité suffisamment élevée pour former l'anion hétérocyclique, à une température généralement comprise entre 10 et 50°C, de préférence vers 25°C. Comme agents anionisants on utilise de préférence l'hydrure de sodium ou l'hydrure de potassium.

La chlorométhylpyridine peut être préparée selon la méthode décrite dans le procédé b.

Procédé d

Les composés selon la formule (I) pour lesquels $R_4$ représente un radical alkoxycarbonylméthyle, comportant de 3 à 8 atomes de carbone et qui répondent à la formule (X) :

$$R\text{-}CH_2\text{-}COOR_6 \qquad (X)$$

dans laquelle R a la même signification que dans la formule (II) et $R_6$ représente un radical alkyle comportant de 1 à 6 atomes de carbone peuvent être obtenus par action de l'alcanol inférieur de formule $R_6OH$ dans laquelle $R_6$ a la même signification que précédemment sur la cyanométhylpyridine de formule (XI) :

$$R\text{-}CH_2\text{-}CN \qquad (XI)$$

dans laquelle R a la même signification que précédemment.

La réaction s'effectue avantageusement en présence d'un acide minéral dans des mélanges solvants susceptibles de dissoudre les réactifs en présence, tels que par exemple les alcools inférieurs, à une température généralement voisine de la température ambiante. La cyanométhylpyridine (XI) peut être obtenue par action du cyanure de sodium sur la chlorométhylpyridine répondant à la formule (VIII). Cette réaction s'effectue avantageusement en milieu hydro-alcoolique dès la température ordinaire. La chlorométhylpyridine (VIII) peut être préparée selon la méthode décrite plus haut dans le procédé b.

Procédé e

Les composés selon la formule (I), pour lesquels $R_4$ représente un radical hydroxyalkyle, c'est à dire les composés répondant à la formule $R\text{-}R_9$ dans laquelle R a la même signification que dans la formule (II) et $R_9$ représente un radical hydroxyalkyle comportant de 2 à 5 atomes

de carbone, peuvent être préparés selon l'un ou l'autre des procédés décrits ci-après :

Les composés selon la formule (I) pour lesquels $R_4$ représente le radical bêta-hydroxyéthyle, et qui répondent donc à la formule (XII) :

$$R-CH_2-CH_2-OH \qquad (XII)$$

dans laquelle R a la même signification que dans la formule (II), peuvent être obtenus à partir de l'alkoxycarbonylméthylpyridine (X), dont la préparation est décrite dans le procédé d, par réduction de ce composé pour donner l'alcool primaire correspondant.

Cette transformation peut être effectuée selon les méthodes usuelles permettant de réduire les esters carboxyliques en alcools, par exemple par hydrogénation catalytique, généralement à température élevée, ou sous pression élevée, ou par utilisation d'un agent réducteur tel que l'aluminohydrure de lithium.

Avantageusement cette réduction est effectuée au moyen de l'aluminohydrure de lithium, en opérant en milieu anhydre, dans un solvant organique inerte tel que les éthers, à une température généralement comprise entre 0 et 30°C, de préférence de l'ordre de 15°C.

Les composés selon la formule (I) pour lesquels $R_4$ représente un radical alpha-hydroxyalkyle, et qui répondent donc à la formule (IX) :

$$R - \underset{}{\overset{OH}{\overset{|}{CH}}} - R_8 \qquad (IX)$$

dans laquelle R a la même signification que dans la formule (II) et $R_8$ représente un radical alkyle comportant de 1 à 4 atomes de carbone, peuvent être obtenus par réduction d'une alcanoylpyridine répondant à la formule (XVIII) :

$$R - CO - R_8 \qquad (XVIII)$$

dans laquelle R et $R_8$ ont la même signification que précédemment.

Cette réduction s'effectue en opérant selon les mêmes conditions que la réduction de l'alkoxycarbonylméthylpyridine (X) pour donner le composé (XII).

L'alcanoylpyridine de départ peut être préparé selon la méthode décrite dans le procédé a.

Les dérivés hydroxyalkyles répondant aux formules (XII) et (IX) dans lesquels R et $R_8$ ont la même signification que précédemment peuvent être transformés par des méthodes connues en d'autres composés répondant à la formule $R-R_9$, définie ci-dessus, par exemple par remplacement du groupe hydroxy par un groupe hydroxyméthyle en opérant selon la succession d'opérations suivante :

- remplacement du groupe hydroxy par un atome de chlore en opérant selon la méthode décrite dans le procédé f,
- remplacement de l'atome de chlore par un groupe alkoxycarbonyle en utilisant la méthode en 2 étapes décrite dans le procédé d,
- réduction du groupe alkoxycarbonyle en groupe hydroxyméthyle en opérant selon la méthode décrite dans le procédé e.

Procédé f

Les composés selon la formule (I) pour lesquels $R_4$ représente un radical halogénoalkyle comportant de 2 à 5 atomes de carbone, c'est à dire les composés de formule $R-R_{10}$ dans laquelle R a la même signification que dans la formule (II) et $R_{10}$ représente un radical halogénoalkyle comportant de 2 à 5 atomes de carbone, peuvent être obtenus par halogénation des hydroxyalkylpyridines de formule $R-R_9$ dont la préparation a été décrite dans le procédé e.

Ainsi lorsque l'hydroxyalkylpyridine répond à la formule (XII) ou à la formule (IX), on obtient respectivement une halogénoalkylpyridine répondant à l'une ou à l'autre des formules (XIII) et (XIX) ci-après :

$$R-CH_2-CH_2-X \qquad (XIII)$$

$$\begin{matrix} X \\ | \\ R - CH - R_8 \end{matrix} \qquad (XIX)$$

dans laquelle R a la même signification que dans la formule (II), $R_8$ a la même signification que dans la formule (XVIII) et X représente un atome d'halogène, de préférence l'atome de chlore.

Cette transformation peut être effectuée selon les méthodes usuelles permettant de remplacer le groupe hydroxyle par un atome d'halogène, par exemple au moyen de chlorures d'oxacides minéraux tels que $SOCl_2$ ou $POCl_3$.

Avantageusement cette halogénation est effectuée au moyen de $SOCl_2$, en opérant dans un solvant organique tel que le chlorure de méthylène, à une température de l'ordre de 10 à 40°C environ.

Procédé q

Les composés selon la formule (I) pour lesquels $R_4$ représente un radical alkyle comportant de 1 à 4 atomes de carbone, et qui répondent donc à la formule (XIV) :

$$R-(C_pH_{2p+1}) \qquad (XIV)$$

dans laquelle R a la même signification que dans la formule (II) et p est égal à un entier de 1 à 4, peuvent être obtenus par réduction de la chloroalkylpyridine répondant à la formule (XVI) :

$$R-(C_pH_{2p})-Cl \qquad (XVI)$$

dans laquelle R et p ont la même signification que précédemment, au moyen d'un réducteur permettant le remplacement de l'atome de chlore par un atome d'hydrogène.

Avantageusement, cette transformation est effectuée en utilisant comme agent réducteur le borohydrure de sodium, en opérant dans un solvant inerte aprotique polaire tel que le diméthylformamide ou le diméthylacétamide, à une température de l'ordre de 20 à 50°C. La chloroalkylpyridine de formule XVI peut être préparée selon la méthode décrite plus haut dans le procédé b, ou selon le procédé f.

<u>Procédé h</u>

Les composés selon la formule (I) pour lesquels $R_4$ représente le radical vinyle peuvent être obtenus par déshydrochloration de la chloroéthylpyridine (XIII) dont la préparation est décrite dans le procédé f.

Cette élimination de HCl s'effectue avantageusement par action de l'hydrure de sodium, en présence de l'anion du triazole-1,2,4 agissant comme agent d'élimination dans un solvant aprotique polaire tel que le diméthylformamide, à température ambiante.

Procédé i

Les composés selon la formule (I) dans laquelle $R_4$ représente un radical alcynyloxyalkyle, et qui répondent donc à la formule (XV) :

$$R-(C_pH_{2p})-O-R_7 \qquad (XV)$$

dans laquelle R a la même signification que dans la formule (II), $R_7$ représente un radical alcynyle comportant de 3 à 5 atomes de carbone et p a la même signification que dans la formule (XIV), peuvent être obtenus par action du sel de métal alcalin de l'alcool $R_7$-OH, ledit sel étant éventuellement formé in situ, sur la chloroalkylpyridine de formule (XVI) dont la préparation est décrite plus haut dans le procédé g. La réaction s'effectue avantageusement dans le tétrahydrofuranne anhydre à environ 20°C.

Les exemples ci-après, donnés à titre non limitatif, illustrent l'invention et montrent comment elle peut être mise en oeuvre.

Les composés répondant à la formule générale (I) peuvent si désiré être transformés, en sels acceptables en agriculture par réaction avec des quantités stoechiométriques d'acides appropriés, tels que ceux mentionnés plus haut.

La structure des composés a été confirmée par spectrométrie infra-rouge et/ou par spectrométrie de résonance magnétique nucléaire (RMN) ; les spectres RMN ont été réalisés à 60 mégahertz dans le diméthylformamide avec l'hexaméthyldisiloxane comme étalon de référence.

L'expression "lutidine-2,6" utilisée dans ces exemples est synonyme de "diméthyl-2,6 pyridine".

Exemple 1 - Préparation de la (S)-N-alpha-méthylbenzyl)-carbamoyl-3 acétyl-5 lutidine-2,6 (composé 1), de formule

n°1

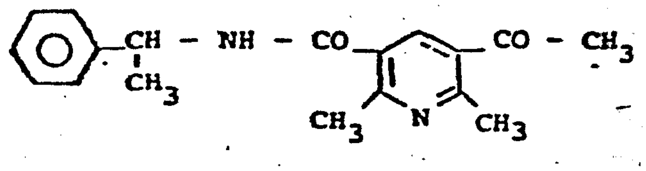

Dans un ballon tricol de 100 ml muni d'une agitation mécanique centrale, d'une ampoule à addition, d'un réfrigérant et d'un thermomètre, on charge 480 mg de magnésium (20 millimoles). On y coule 3 g d'iodure de méthyle (21 millimoles) dans 25 ml d'éther éthylique anhydre et agite jusqu'à la disparition du magnésium. Après refroidissement à 25°C, on coule 2,2 g (7 millimoles) de

(S)-N-(alpha-méthylbenzyl)-carbamoyl-3 méthoxycarbonyl-5 lutidine-2,6 dans 25 ml de tétrahydrofuranne anhydre. La réaction est exothermique. La température du milieu monte jusqu'à 45°C. Après une heure d'agitation, le milieu est traité par 50 ml d'une solution aqueuse à 5 % de chlorure d'ammonium.

La phase organique est décantée, lavée à l'eau, séchée sur sulfate de sodium et concentrée. Après chromatographie sur 200 g de silice éluée par le mélange acétate d'éthyle-hexane en proportions égales, on obtient 1,25 g du produit attendu (composé n°1) sous la forme d'une poudre blanche.

Rendement : 60 %

Point de fusion : 128°C

Spectre IR : fréquence du groupe carbonyle : 1693 $cm^{-1}$

La (S)-N-(alpha-méthylbenzyl) carbamoyl-3 méthoxycarbonyl-5 lutidine-2,6, utilisée comme produit de départ a été obtenue par action de $KMnO_4$ sur la (S)-N-(alpha-méthylbenzyl) carbamoyl-3 méthoxycarbonyl-5 diméthyl-2,6 dihydro-1,4 pyridine, elle même obtenue par réaction du formaldéhyde avec un dérivé aminoéthylénique et une N-acétylamide appropriés, selon un procédé analogue à celui décrit dans le brevet européen 0.044262, dans le cas de la phénylcarbamoyl-3 méthoxycarbonyl-5 dihydro-1,4 lutidine-2,6.

Exemple 2

En opérant selon les conditions indiquées dans l'exemple précédent à partir de (S)-N-(alpha-méthylbenzyl)-carbamoyl-3 méthoxycarbonyl-5 lutidine-2,6 et d'iodure d'éthylmagnésium, on a préparé la (S)-N-(alpha-méthylbenzyl)-carbamoyl-3 propionyl-5 lutidine-2,6 (composé n°2) de formule

n°2

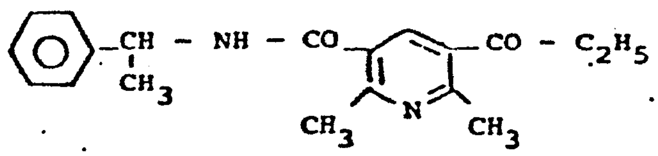

Rendement : 47 %
Point de fusion : 120°C

Spectre IR : fréquence du groupe carbonyle : 1695 $cm^{-1}$.

<u>Exemple 3 : Préparation de la (S)-N-(alpha-méthylbenzyl)-carbamoyl-3 azidométhyl-5 lutidine-2,6 (composé n°3)</u> de formule

n°3

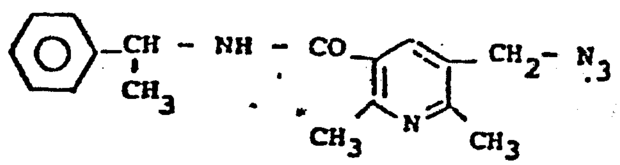

Dans un ballon tricol de 2 l muni d'une ampoule à addition, d'une agitation mécanique centrale, d'un réfrigérant surmonté d'un tube de garde à $CaCl_2$, d'un thermomètre, on charge 500 ml de tétrahydrofuranne anhydre. Par petites portions, on y ajoute 14 g d'hydrure de lithium et d'aluminium. Le milieu réactionnel est refroidi à 0°C. On y coule lentement, entre 0 et 10°C, une solution de 50 g (160 millimoles) de (S)-N-(alpha-méthylbenzyl)-carbamoyl-3 méthoxycarbonyl-5 lutidine-2,6 dans 300 ml de tétrahydrofuranne anhydre. Après la fin de l'addition, le milieu réactionnel est réchauffé jusqu'à 30°C et agité pendant 45 minutes à cette température.

Après hydrolyse et extraction, on obtient 45 g d'un produit jaune qui est purifié par recristallisation dans 300 ml d'acétate d'éthyle. On obtient 42 g de (S)-N-(alpha-méthylbenzyl)-carbamoyl-3 hydroxyméthyl-5 lutidine 2,6 de formule :

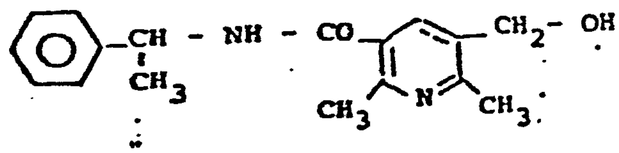

Rendement : 90 %

Point de fusion : 143°C

Spectre I.R : 3400 $cm^{-1}$, 3250 $cm^{-1}$ et 3150 $cm^{-1}$ (groupe OH)

Spectre RMN : Déplacement chimique des groupes méthyle 2,40 ppm et 2,70 ppm.

Dans un ballon tricol de 4 l muni d'une ampoule à addition, d'une agitation mécanique centrale, d'un réfrigérant et d'un thermomètre, on coule 1800 ml de chlorure de méthylène. On y ajoute 90 g (317 millimoles) du composé obtenu dans l'étape précédente, c'est-à-dire la (S)-N-(alpha-méthylbenzyl)-carbamoyl-3 hydroxyméthyl-5 lutidine-2,6. Après dissolution, on coule lentement 73 g (613 millimoles) de chlorure de thionyle. La réaction est faiblement exothermique, le milieu réactionnel est maintenu à reflux pendant 30 mn à partir de la fin du dégagement gazeux.

Après refroidissement, le milieu est neutralisé par 380 ml de soude 2N. La phase organique est décantée, lavée à l'eau et sèchée sur sulfate de sodium. On obtient ainsi, après concentration, 90 g de (S)-N-(alpha-méthylbenzyl)-carbamoyl-3 chlorométhyl-5 lutidine 2,6 de formule

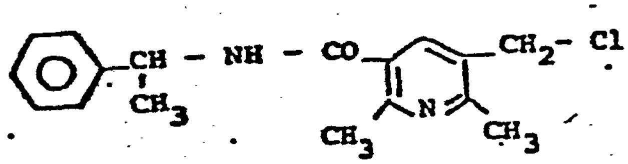

Point de fusion : 165°C

Rendement : 94 %

Spectre RMN : Déplacements chimiques des groupes méthyle 2,50 et 2,75 ppm.

Dans un Erlenmeyer de 250 ml, on charge 3,7 g (12 millimoles) du composé obtenu dans l'étape précédente, c'est-à-dire la (S)-N-(alpha-méthylbenzyl)-carbamoyl-3 chlorométhyl-5 lutidine-2,6 et 120 ml d'alcool éthylique. Après dissolution, on y ajoute une solution de 1 g d'azoture de sodium (15 millimoles) dans 20 ml d'eau. Après 8 h, le milieu réactionnel est versé dans 150 ml d'eau et extrait par 2 fois 100 ml de chlorure de méthylène. La phase organique est lavée à l'eau, séchée sur sulfate de sodium et concentrée. On obtient ainsi 3,2 g de produit brut.

Après filtration sur 100 g de silice par le mélange chlorure de méthylène-acétone en proportions équivalentes, on obtient 3 g du produit attendu (composé n°3) : Sous la forme d'une poudre blanche fondant à 124-125°C.

Rendement : 80%

Spectre I.R : 2075, 2095 et 2112 $cm^{-1}$ (substituant azido)

<u>Exemple 4 : Préparation de la (S)-N-(alphaméthylbenzyl)-carbamoyl-3 (triazolyl-1,2,4)-méthyl-5 lutidine-2,6 (composé n°4)</u> de formule :

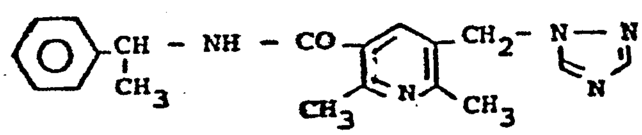

n°4

Dans un ballon tricol de 250 ml muni d'une ampoule à addition, d'une agitation mécanique centrale et d'un réfrigérant surmonté d'un tube de garde à chlorure de calcium, on charge 20 ml de DMF et 2 g (6,6 millimoles) de (S)-N-(alpha-méthylbenzyl)-carbamoyl-3 chlorométhyl-5 lutidine-2,6 dont la préparation a été décrite dans l'exemple 3.

Après dissolution on y ajoute 480 mg de triazole-1,2,4 et 500 mg (16,6 millimoles) d'hydrure de sodium à 80 %

Lorsque la réaction est achevée, le milieu réactionnel est versé dans 200 ml d'eau et extrait par deux fois 50 ml de chlorure de méthylène. Après lavage à l'eau, séchage sur sulfate de sodium et concentration de la phase organique, on obtient 1,8 g du produit attendu (composé n°4)
Point de fusion : 145°C
Rendement : 81 %
Spectre RMN : Déplacements chimiques des groupes méthyle 2,42 et 2,64 ppm

<u>Exemple 5 : Préparation de la (S)-N-(alphaméthylbenzyl)-carbamoyl-3 (éthoxycarbonylméthyl-5 lutidine-2,6 (composé n°5)</u> de formule :

n°5

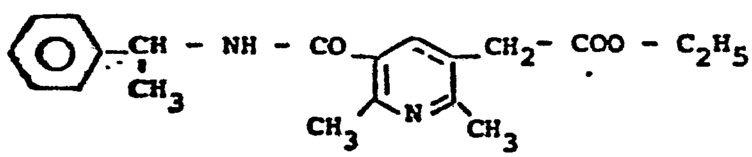

Dans un ballon monocol de 500 ml agité magnétiquement, on coule 100 ml d'éthanol absolu. On refroidit dans la glace et coule lentement 80 ml d'acide sulfurique puis 5 ml d'eau et 6,4 g (22 millimoles) de (S)-N-(alpha-méthylbenzyl)-carbamoyl-3 cyanométhyl-5 lutidine-2,6.

Après 90 mn à reflux, le milieu réactionnel est refroidi et traité par 110 g de bicarbonate de sodium (1,3 mole) dans un litre d'eau. On extrait par 3 fois 100 ml de chlorure de méthylène. Après lavage à l'eau, la phase organique est séchée et concentrée. Le produit obtenu est chromatographié sur 150 g de silice. Le mélange acétone-hexane en proportions équivalentes élue 4 g du produit attendu (composé n°5)
Rendement : 54 %
Point de fusion : 135°C
La (S)-N-(alpha-méthylbenzyl)-carbamoyl-3 cyanométhyl-5 lutidine-2,6 a été obtenue par action du cyanure de sodium

sur la (S)-N-(alpha-méthylbenzyl)-carbamoyl-3 chlorométhyl-5 lutidine-2,6.

Exemple 6 : Préparation de la (S)-N-(alpha-méthylbenzyl)-carbamoyl-3 bétahydroxyéthyl-5 lutidine-2,6 (composé n°6) de formule :

n°6 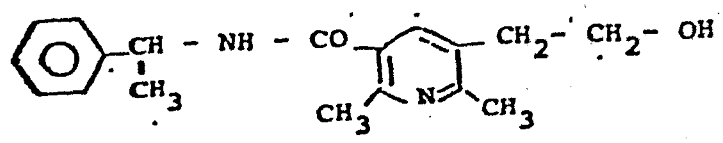

Dans un ballon tricol de 1 l muni d'une agitation mécanique centrale, d'une ampoule à addition, d'un réfrigérant ascendant surmonté d'un tube de garde à chlorure de calcium, on coule 250 ml de tétrahydrofuranne anhydre. On charge ensuite, en agitant, 4,6 g (0,12 mole) d'hydrure de lithium et d'aluminium. Puis on coule, entre 5 et 10°C, une solution de 21 g (62 millimoles) de (S)-N-(alpha-méthylbenzyl)-carbamoyl-3 éthoxycarbonylméthyl-5 lutidine-2,6 (composé n°5), dans 150 ml de tétrahydrofuranne anhydre. L'addition dure 2 heures. Après la fin de l'addition, on agite pendant 30 mn supplémentaires à 30°C.

Après refroidissement et hydrolyse, la phase organique est séchée et concentrée. Le produit obtenu est dissous à ébullition dans 500 ml de toluène. Après refroidissement et filtration, on obtient 16,3 g du produit attendu (composé n°6)

Point de fusion : 116°C

Rendement : 88 %

Exemple 7 : Préparation de la (S)-N-(alpha-méthylbenzyl)-carbamoyl-3 bêtachloroéthyl-5 lutidine-2,6 (composé n°7) de formule :

n°7 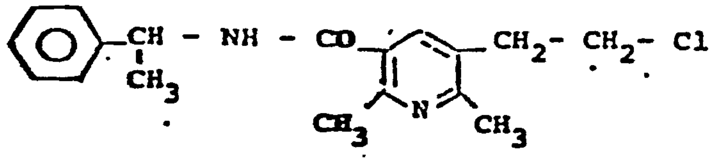

Dans un ballon tricol de 1 l muni d'une agitation mécanique centrale, d'un réfrigérant et d'un thermomètre , on coule 500 ml de chlorure de méthylène et charge 15,2 g (51 millimoles) de (S)-N-(alpha-méthylbenzyl)-carbamoyl-3 bêtahydroxyéthyl-5 lutidine-2,6 (composé n°6) dont la préparation est décrite dans l'exemple 6.

On chauffe à reflux jusqu'à dissolution, refroidit vers 30°C et ajoute 10,3 g (76 millimoles) de chlorure de thionyle. Après 2 h d'agitation à cette température, on neutralise en coulant lentement 300 ml d'une solution aqueuse saturée en bicarbonate de sodium. La phase organique est décantée, lavée à l'eau et séchée sur sulfate de sodium. Après concentration, le produit brut est recristallisé dans le toluène. On obtient 7,5 g du produit attendu (composé n°7)

Rendement : 46 %

Point de fusion : 125°C

Spectre RMN : Déplacements chimiques des deux méthyles : 2,36 et 2,68 ppm.

<u>Exemple 8 : Préparation de la (S)-N-(alpha-méthylbenzyl)-carbamoyl-3 éthyl-5 lutidine-2,6 (composé n°8)</u> de formule :

n°8 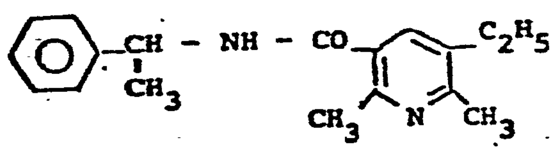

Dans un ballon monocol de 250 ml, on dissout 3,4 g (11 millimoles) de (S)-N-(alpha-méthylbenzyl)-carbamoyl-3 bêtachloroéthyl-5 lutidine-2,6 (composé n°7) dans 20 ml de diméthylformamide. On ajoute ensuite 1 g (26 millimoles) de borohydrure de sodium. Après 4 h d'agitation à 50°C, on refroidit à 20°C et ajoute une solution de 5 g de chlorure d'ammonium dans 100 ml d'eau. On extrait au chlorure de méthylène. Après le traitement habituel, le produit obtenu est chromatographié sur 100 g de silice. L'acétate d'éthyle élue 1,7 g du produit attendu (composé n°8) .

Point de fusion : 123°C

Rendement : 61 %

Spectre RMN : Déplacement chimique des trois méthyles : 0,76-2,35 et 2,70 ppm.

<u>Exemple 9</u>

En opérant selon la méthode décrite dans l'exemple n°8, à partir de la (S)-N-(alpha-méthylbenzyl)-carbamoyl-3 chlorométhyl-5 lutidine-2,6, dont la préparation est décrite dans l'exemple n°3, on a préparé la (S)-N-(alpha-méthylbenzyl)-carbamoyl-3 triméthyl-2,5,6 pyridine (composé n°9) de formule :

n°9

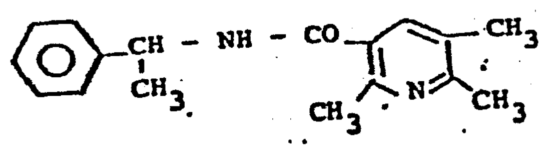

Point de fusion : 136°C

Rendement : 87 %

Spectre RMN : Déplacements chimiques des trois méthyles : 1,83 - 2,26 et 2,69 ppm.

<u>Exemple 10 : Préparation de la (S)-N-(alpha-méthylbenzyl)-carbamoyl-3 vinyl-5 lutidine-2,6 (composé n°10)</u> de formule :

n°10 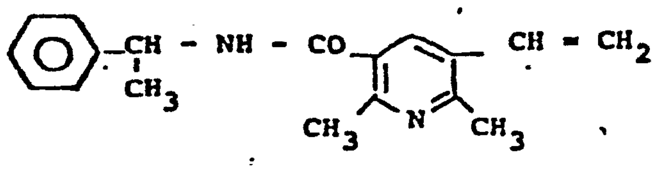

Dans un ballon monocol de 100 ml, on charge 25 ml de diméthylformamide et 1,2 g (17 millimoles) de triazole-1,2,4. On y ajoute 700 mg d'hydrure de sodium à 80 % (23 millimoles) et 2 g (6 millimoles) de (S)-N-(alpha-méthylbenzyl)-carbamoyl-3 bétachloroéthyl-5 lutidine-2,6 (composé n°7).

Après 8 h d'agitation à température ambiante, le milieu réactionnel est versé dans 200 ml d'eau et extrait par deux fois 100 ml de chlorure de méthylène. Après décantation la phase organique est lavée à l'eau est séchée sur sulfate de sodium. Le produit obtenu après concentration est chromatographié sur 100 g de silice. Le mélange acétate d'éthyle-hexane en proportions équivalentes élue 1 g du produit attendu (composé n°10).

Point de fusion : 121°C

Rendement : 56 %

Spectre RMN : Déplacements chimiques des deux méthyles : 2,36 ppm et 2,89 ppm

<u>Exemple 11 :Préparation de la (S)-N-(alpha-méthylbenzyl)-carbamoyl-3 propargyloxyméthyl-5 lutidine-2,6 (composé n°11)</u> de formule

n°11 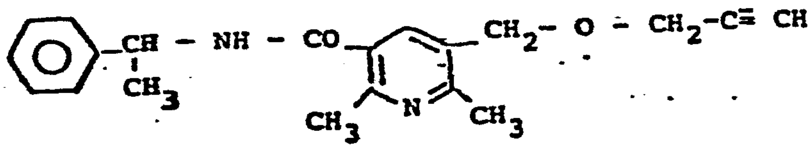

Dans un ballon monocol de 500 ml, on charge 8 g d'alcool propargylique (143 millimoles) et 130 ml de tétrahydrofuranne anhydre. En refroidissant à 20°C, on y ajoute lentement 4 g (133 millimoles) d'hydrure de sodium à 80 % . Puis on y coule une solution de 6 g (20 millimoles) de (S)-N-(alpha-méthylbenzyl)-carbamoyl-3 chlorométhyl-5 lutidine-2,6, dans 50 ml de tétrahydrofuranne. Après 24 h d'agitation à température ambiante, le milieu réactionnel est versé dans 500 ml d'eau et neutralisé par 30 ml d'acide chlorhydrique 4N. On extrait par 2 fois 100 ml de chlorure de méthylène. La phase organique est lavée à l'eau, séchée et concentrée. On obtient ainsi 4 g d'une huile qui est chromatographiée sur 200 g de silice. Le mélange chlorure de méthylène/acétone 8/2 élue 2,1 g du produit attendu (composé n°11)

Point de fusion : 105°C

Rendement : 33 %

Spectre RMN : Déplacement chimique des 2 méthyle : 2,39 et 2,71 ppm.

<u>Exemple 12</u> : <u>Préparation de la (S)-N-(alpha-méthylbenzyl)carbamoyl-3 (chloro-1)-propyl-3 lutidine-2,6 (composé n° 12) de formule</u> :

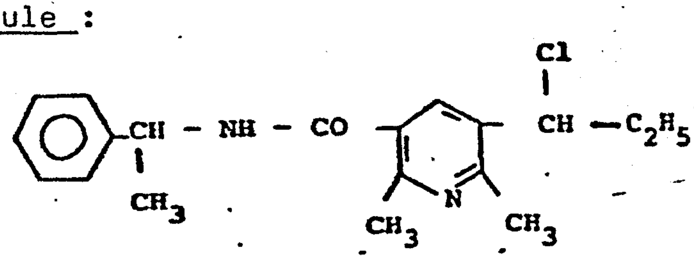

En opérant selon la méthode décrite dans l'exemple 6, à partir de 6,2 g de (S)-N-(alpha-méthylbenzyl)-carbamoyl-3 éthylcarbonyl-2,6 (composé n° 2), et de 1,6 g d'hydrure de lithium et d'aluminium, on obtient 5,2 g de (S)-N-(alpha-méthylbenzyl)-carbamoyl-3 (hydroxy-1) propyl-3 lutidine-2,6 - Rendement : 83 %.

En opérant selon la méthode décrite dans l'exemple 7, à partir de 5,2 g de ce composé et de 2,7 g de chlorure de thionyle, on obtient 2,7 g du produit attendu (composé n° 12) - Rendement : 48 %.

<u>Exemple n° 13</u> : <u>Préparation du chlorhydrate du (S)-N-(alpha-méthylbenzyl)-carbamoyl-3 azidométhyl-5 lutidine-2,6</u> (composé n° 13 - chlorhydrate du composé n° 3)

On agite jusqu'à dissolution d'une suspension de 22 g de composé n° 3 dans 102 ml d'acide chlorhydrique à 0,7 N. Après dissolution, on évapore le solvant sous pression réduite. On obtient 2,4 d'un produit cristallisé fondant à 165°C. Rendement : 97 %

<u>Exemple n° 14</u> : <u>Préparation du sulfate du (S)-N-(alpha-méthylbenzyl)-carbamoyl-3 propionyl-5 lutidine-2,6</u> (composé n° 14 - sulfate du composé n° 2)

On dissout 3,1 g de composé n° 2 dans 200 ml d'acétone et on ajoute 110 ml d'une solution 2N d'acide sulfurique dans l'éther éthylique. Après 30 minutes, on filtre le précipité et on obtient 3,1 g du produit attendu. Rendement : 76 %.

Point de fusion : 155 °C.

<u>Exemple 15 : Application herbicide, en prélevée des espèces végétales</u>.

Dans des pots de 9 x 9 x 9 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur.

Après humidification de la terre, les pots sont traités par pulvérisation de bouillie en quantité correspondant à une dose d'application de 500 l/ha et contenant la matière active à la concentration considérée.

La bouillie a été préparée en diluant par un égal volume de solution aqueuse à 1 g/l de Cémulsol NP10 une solution de concentration voulue du produit à tester dans le mélange suivant :

| | | |
|---|---|---|
| Soprophor FL | | 15 g/l |
| Sapogenat TO 80 | | 3 g/l |
| Diméthylformamide | q.s.p. | 1000 ml |

Le Cémulsol NP10 est un agent tensio-actif non ionique constitué d'alkylphénols polyéthoxylés, principalement de nonylphénol polyéthoxylé. Le Soprophor FL est un agent tensio-actif anionique constitué par des esters phosphoriques d'alcools ou phénols polyoxyéthylés.

Le Sapogenat TO 80 est un agent tensio-actif non ionique constitué par des trialkylphénols.

Selon la concentration en matière active de la bouillie, la dose de matière active appliquée a été de 1 Kg/ha à 8 kg/ha.

Les pots traités sont ensuite placés dans des bacs destinés à recevoir l'eau d'arrosage, en subirrigation, et maintenus pendant 28 jours à température ambiante sous 70 % d'humidité relative.

Au bout de 28 jours, on compte le nombre de plantes vivantes dans les pots traités par la bouillie contenant la matière active à tester et le nombre de plantes vivantes dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100 % indique qu'il y a eu destruction complète de l'espèce végétale considérée et un pourcentage de 0 % indique que le nombre de plantes vivantes dans le pot traité est identique à celui dans le pot témoin.

Pour les essais de cet exemple, les espèces végétales utilisées ont été les suivantes :

| Adventices monocotylédones : | Abréviation utilisée |
| --- | --- |
| Folle avoine (Avena fatua) | FAV |
| Panisse (Echinochloa crus-galli) | PAN |
| Ray-grass (Lolium multiflorum) | RAY |
| Adventices dicotylédones : | |
| Chénopode (Chenopodium sp) | CHE |
| Moutarde (Sinapis arvensis) | MOU |
| Cultures dicotylédones : | |
| Haricot (Phaseolus vulgaris) | HAR |

Les résultats observés sont indiqués dans le tableau I ci-après.

Le produit de comparaison est la N-(phényl)carbamoyl-pyridine, non substituée sur le phényle, décrite en tant que composé n° 25 de la demande de brevet européen n° 0 044 262, et dont le nom chimique est : N-(phényl)-carbamoyl-3 éthoxy carbonyl-5 lutidine-2,6.

Exemple 16 : Application herbicide, en postlevée des espèces végétales.

Dans des pots de 9 x 9 x 9 cm remplis de terre agricole légère, on sème un nombre de graines déterminé en fonction de l'espèce végétale et de la grosseur de la graine.

On recouvre ensuite les graines d'un couche de terre d'environ 3 mm d'épaisseur et on laisse germer la graine jusqu'à ce qu'elle donne naissance à une plantule de 5 à 10 cm de hauteur.

Les pots sont alors traités par pulvérisation de bouillie en quantité correspondant à une dose d'application de 500 l/ha et contenant la matière active à la concentration considérée.

La bouillie a été préparée de la même manière qu'à l'exemple 15.

Selon la concentration en matière active de la bouillie, la dose de matière active appliquée a été de 1 à 8 kg/ha.

Les pots traités sont ensuite placés dans des bacs destinés à recevoir l'eau d'arrosage, en subirrigation, et maintenus pendant 28 jours à température ambiante sous 70 % d'humidité relative.

Au bout de 28 jours, on compte le nombre de plantes vivantes dans les pots traités par la bouillie contenant la matière active à tester et le nombre de plantes vivantes dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100 % indique qu'il y a eu destruction complète de l'espèce végétale considérée et un pourcentage de 0 % indique que le nombre de plantes vivantes dans le pot traité est identique à celui dans le pot témoin.

Le nom et l'abréviation des espèces végétales utilisées sont tels qu'indiqués précédemment.

Les résultats observés sont indiqués dans le tableau (II)

Exemple 17 : Sélectivité vis à vis des cultures, en prélevée des espèces végétales :

On procède selon la méthode décrite dans l'exemple 15, en remplaçant les espèces végétales par les cultures suivantes :

- coton (*Gossypium barbadense*)
- soja (*glycine Max*)

et en effectuant le traitement à la dose dé 1kg/ha.

Dans ces conditions, on a observé qu'à cette dose :

- les composés n° 1, 8, 9 et 11 sont bien tolérés par le coton,
- les composés n° 1, 3, 7, 8 et 11 sont bien tolérés par le soja.

Les résultats décrits dans ces exemples 15 à 17 montrent l'excellente activité herbicide des composés selon l'invention sur la plupart des adventices traitées, tant graminées que dicotylédones.

Pour leur emploi pratique, les composés selon l'invention sont rarement utilisés seuls. Le plus souvent ils font partie de compositions. Ces compositions, utilisables comme agents herbicides, contiennent comme matière active un composé selon l'invention tel que décrit précédemment en association avec les supports solides ou liquides, acceptables en agriculture et les agents tensio-actifs également acceptables en agriculture. En particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides, fongicides ou herbicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Les doses d'emploi des composés selon l'invention peuvent varier dans de larges limites, notamment selon la nature des adventices à éliminer et le degré d'infestation habituel des cultures pour ces adventices.

D'une façon générale, les compositions selon l'invention contiennent habituellement de 0,05 à 95 % environ (en poids) d'un ou plusieurs composés selon l'invention, de 1 % à 94,95 % environ de un ou plusieurs supports solides ou liquides et éventuellement de 0,1 à 20 % environ de un ou plusieurs agents tensioactifs.

Selon ce qui a déjà été dit les composés selon l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des al ylphénols ou des arylphénols), des sels d'esters d'acides sulfosucciniques, des dérivés de la taurine (notamment des al yltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les

granulés, notamment ceux obtenus par extrusion, par compactage, par imprégnation d'un support granulé, par granulation à partir d'une poudre (la teneur en composé de formule (I) dans ces granulés étant entre 0,5 et 80 % pour ces derniers cas).

Comme formes de compositions liquides ou destinées à constituer des compositions liquides lors de l'application, on peut citer les solutions, en particulier les concentrés solubles dans l'eau et les concentrés émulsionnables, les émulsions, les suspensions concentrées (ou flowables), les poudres mouillables (ou poudre à pulvériser) et les pâtes.

Les concentrés émulsionnables contiennent la matière active dissoute dans un solvant, habituellement un hydrocarbure aromatique et éventuellement en utilisant un co-solvant qui peut être par exemple une cétone, un ester, un éther-oxyde etc... Ils contiennent habituellement de 10 à 60 % en poids/volume de matière active et de 2 à 20 % en poids/volume d'agent émulsifiant. Ils peuvent également contenir, si nécessaire, divers additifs appropriés tels que des agents tensioactifs, des stabilisants, des agents de pénétration, des inhibiteurs de corrosion, des colorants, des adhésifs etc ...

Les suspensions concentrées (ou flowables), qui sont applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des antimousses, des inhibiteurs de corrosion, des stabilisants, des agents de pénétration et des adhésifs et, comme support, de l'eau ou un liquide organique dans lequel la matière active est peu soluble ou non soluble : certaines matières solides organiques ou des sels minéraux peuvent être dissous dans le support pour aider à empêcher la sédimentation ou comme antigels pour l'eau.

A tire d'exemple voici la composition d'une suspension concentrée :

- matière active ............................ 250 g
- alkylphénol polyéthoxylé par 10 motifs oxyde d'éthylène (agent mouillant) ........ 10 g
- phosphate de polyaryl éthoxylé et salifié (dispersant) ............................ 10 g
- propylène-glycol (antimousse) ............. 50 g
- polysaccharide (agent épaississant) ....... 2 g
- eau Q.S.P.................................. 1 l

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

- matière active ................................ 50 %
- lignosulfonate de calcium (défloculant) ........ 5 %
- isopropylnaphtalène sulfonate (agent mouillant anionique) ............................ 1 %
- silice antimottante ........................... 5 %
- kaolin (charge) ............................... 39 %

Un autre exemple de poudre mouillable à 80 % est donné ci-après :

- matière active ................................ 80 %
- alkylnaphtalène sulfonate de sodium ............ 2 %
- lignosulfonate de sodium ....................... 2 %
- silice antimottante ............................ 3 %
- kaolin......................................... 13 %

Un autre exemple de poudre mouillable est donné ci-après :

- matière active .................................. 50 %
- alkylnaphtalène sulfonate de sodium ............. 2 %
- méthyl cellulose de faible viscosité ............ 2 %
- terre de diatomées .............................. 46 %

Un autre exemple de poudre mouillable est donné ci-après :

- matière active .................................. 90 %
- dioctylsulfosuccinate de sodium ................. 0,2 %
- silice synthétique .............................. 9,8 %

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions, par exemple les compositions obtenues en diluant à l'aide d'eau une poudre mouillable selon l'invention, sont comprises dans le cadre général de la présente invention. On utilise le terme de bouillie pour désigner les compositions diluées à l'eau telles qu'on les applique aux cultures.

Toutes ces dispersions ou émulsions aqueuses ou bouillies sont applicables aux cultures à désherber par tout moyen convenable, principalement par pulvérisation, à des doses qui sont généralement de l'ordre de 100 à 1 200 litres de bouillie à l'hectare.

Les granulés destinés à être disposés sur le sol sont habituellement préparés de manière qu'ils aient des dimensions comprises entre 0,1 et 2 mm et ils peuvent être fabriqués par agglomération ou imprégnation. De préférence, les granulés contiennent 1 à 25 % de matière active et 0 à 10 % d'additifs comme des stabilisants, des agents de modification à libération lente, des liants et des solvants.

Selon un exemple de composition de granulé, on utilise les constituants suivants :

- matière active ............................ 50 g
- éther de cétyle et de polyglycol ......... 2,5 g
- polyéthylène glycol ....................... 35 g
- kaolin (granulométrie : 0,3 à 0,8 mm) ... 910 g.

Dans ce cas particulier on mélange la matière active avec l'épichlorhydrine et on disperse dans 60 g d'acétone ; on ajoute alors le polyéthylène glycol et l'éther de cétyle et de polyglycol. On arrose le kaolin avec la dispersion obtenue et on évapore ensuite l'acétone sous vide.

Comme indiqué plus haut, l'invention concerne également un procédé de désherbage de cultures, notamment le coton, et le soja, selon lequel on applique sur les plantes et/ou sur le sol de la zone à désherber une quantité efficace d'au moins un des composés selon l'invention. Ceux-ci sont utilisés pratiquement sous forme des compositions herbicides selon l'invention qui ont été décrites ci-avant. Généralement, des quantités de matière active allant de 0,1 à 3 kg/ha donnent de bons résultats, étant entendu que le choix de la quantité de matière active à utiliser est fonction de l'intensité du problème à résoudre, des conditions climatiques et de la culture considérée. Le traitement est en général effectué en prélevée des cultures et adventices, ou en présemis des cultures avec incorporation dans le sol (cette incorporation est donc une opération complémentaire du procédé de traitement de l'invention), bien que dans certains cas, selon le composé utilisé de bons résultats puissent également être obtenus par des traitements de

post-levée. D'autres modes de mise en oeuvre du procédé de traitement selon l'invention peuvent encore être utilisés : ainsi on peut appliquer la matière active sur le sol, avec ou sans incorporation, avant repiquage d'une culture.

Le procédé de traitement de l'invention s'applique aussi bien dans le cas de cultures annuelles que dans le cas de cultures pérennes ; dans ce dernier cas on préfère appliquer les matières actives de l'invention de manière localisée, par exemple entre les rangs des dites cultures.

TABLEAU (I)

Activité herbicide en serre en prélevée des espèces végétales

% de destruction par rapport au témoin

Espèces végétales

| Composé n° | Dose Kg/ha | FAV | PAN | RAY | HAR | CHE | MOU |
|---|---|---|---|---|---|---|---|
| 1 | 1<br>8 | 100<br>100 | 95<br>100 | 100<br>100 | 0<br>50 | 100<br>100 | 80<br>100 |
| 2 | 1<br>8 | 100<br>100 | 100<br>100 | 100<br>100 | 80<br>100 | 100<br>100 | 100<br>100 |
| 3 | 1<br>8 | 80<br>100 | 100<br>100 | 100<br>100 | 0<br>0 | 100<br>100 | 100<br>100 |
| 4 | 1<br>8 | 0<br>20 | 20<br>80 | 30<br>80 | 100<br>100 | 100<br>100 | 100<br>100 |
| 7 | 1<br>8 | 100<br>100 | 100<br>100 | 100<br>100 | 0<br>100 | 100<br>100 | 100<br>100 |
| 8 | 1<br>8 | 100<br>100 | 100<br>100 | 100<br>100 | 0<br>100 | 100<br>100 | 100<br>100 |
| 9 | 1<br>8 | 95<br>100 | 100<br>100 | 100<br>100 | 0<br>100 | 100<br>100 | 20<br>100 |
| 10 | 1<br>8 | 90<br>100 | 100<br>100 | 100<br>100 | 0<br>0 | 100<br>100 | 80<br>100 |
| 11 | 1<br>6 | 30<br>100 | 100<br>100 | 100<br>100 | 0<br>40 | 100<br>100 | 100<br>100 |
| 12 | 1 | 90 | 100 | 100 | 50 | 100 | 30 |
| Comparaison | 2<br>8 | 0<br>10 | 0<br>60 | 0<br>5 | 0<br>50 | 30<br>100 | 0<br>50 |

Comparaison = EP-A-0 044 262 - Composé n° 25.

TABLEAU (II)

Activité herbicide en serre en postlevée des espèces végétales
% de destruction par rapport au témoin

Espèces végétales

| Composé n° | Dose Kg/ha | FAV | PAN | RAY | HAR | CHE | MOU |
|---|---|---|---|---|---|---|---|
| 1 | 1<br>8 | 100<br>100 | 100<br>100 | 100<br>100 | 0<br>100 | 30<br>20 | 0<br>70 |
| 2 | 1<br>8 | 100<br>100 | 100<br>100 | 100<br>100 | 100<br>100 | 40<br>100 | 60<br>100 |
| 3 | 1<br>8 | 30<br>100 | 100<br>100 | 100<br>100 | 100<br>100 | 30<br>100 | 30 |
| 4 | 1<br>8 | 0<br>20 | 0<br>50 | 20<br>30 | 100<br>100 | 0<br>20 | 30<br>100 |
| 7 | 1<br>8 | 100<br>100 | 100<br>100 | 100<br>100 | 100<br>100 | 0<br>60 | 0<br>100 |
| 8 | 1<br>8 | 95<br>100 | 100<br>100 | 80<br>100 | 100<br>100 | 80<br>100 | 20<br>100 |
| 9 | 1<br>8 | 60<br>100 | 20<br>100 | 30<br>100 | 100<br>100 | 30<br>100 | 20<br>100 |
| 10 | 1<br>8 | 90<br>100 | 40<br>100 | 60<br>100 | 0<br>100 | 40<br>100 | 30<br>95 |
| 11 | 1<br>6 | 20<br>100 | 40<br>100 | 90<br>100 | 100<br>100 | 20<br>50 | 80<br>100 |
| 12 | 1 | 90 | 100 | 90 | 100 | 20 | 20 |
| Comparaison | 8 | 0 | 20 | 0 | 0 | 30 | 20 |

Comparaison = EP-A-0 044 - Composé n° 25.

## REVENDICATIONS

1) - Dérivé de la benzylcarbamoylpyridine caractérisé en ce qu'il répond à la formule :

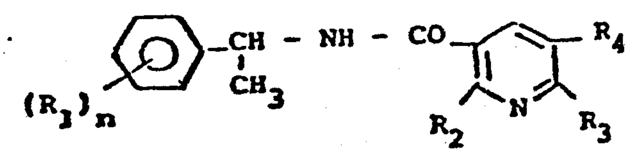

dans laquelle :

$R_1$ représente un atome d'halogène ou un radical alkyle comportant de 1 à 6 atomes de carbone, éventuellement substitué par un ou plusieurs atomes d'halogène, ou un radical alkoxy comportant de 1 à 4 atomes de carbone,

n est un nombre entier de 0 à 5, valeurs limites comprises, étant entendu que lorsque plusieurs substituants $R_1$ sont présents, ces substituants peuvent être soit identiques, soit différents,

$R_2$ et $R_3$, identiques ou différents représentent un radical alkyle comportant de 1 à 3 atomes de carbone ou un radical alkoxy comportant de 1 à 4 atomes de carbone ou un radical alkoxyalkyle comportant de 2 à 8 atomes de carbone,

$R_4$ représente un radical choisi parmi les radicaux acyle ; azidométhyle ; alkoxycarbonylméthyle comportant de 3 à 8 atomes de carbone ; hydroxyalkyle comportant de 2 à 5 atomes de carbone ; halogénoalkyle comportant de 2 à 5 atomes de carbone ; alkyle comportant de 1 à 4 atomes de carbone, méthyle substitué par un radical hétérocyclique comportant de 5 à 6 chaînons et de 1 à 3 hétéroatomes choisis parmi les atomes d'oxygène, de soufre et d'azote ;

vinyle et alcynyloxyalkyle comportant de 4 à 9 atomes de carbone,

et sels acceptables en agriculture de ce dérivé de benzylcarbamoylpyridine,

et formes isomères, optiquement actives, de ce dérivé de benzylcarbamoylpyridine, seules ou en mélanges entre elles.

2) - Composé selon la revendication 1 caractérisé en ce que n est égal à 0 ou à 1 ou à 2 ou à 3,

$R_1$ représente un atome d'halogène ou le radical méthyle,

$R_2$ représente le radical méthyle,

$R_3$ représente le radical méthyle ou méthoxyméthyle,

$R_4$ représente un radical alcanoyle comportant de 1 à 6 atomes de carbone, azidométhyle, halogénoalkyle comportant de 2 à 5 atomes de carbone, ou alkyle comportant de 1 à 3 atomes de carbone,

et sels de ce composé formés avec des acides appropriés,

et formes isomères optiquement actives, seules ou en mélange entre elles.

3) - Composé selon la revendication 2, caractérisé en ce que

n est égal à zéro, $R_2$ et $R_3$ représentent le radical méthyle, $R_4$ représente un radical alcanoyle comportant de 1 à 3 ~~atomes de carbone~~, azidométhyle, halogénoalkyle comportant de 2 à 4 atomes de carbone ou alkyle comportant de 1 à 3 atomes de carbone,

et sels acceptables en agriculture de ce composé,

et formes isomères, optiquement actives, seules ou en mélange entre elles.

4) - Composé selon l'une des revendications 1 à 3, caractérisé en ce qu'il est sous forme d'isomère optique de même configuration que la (S)-alpha-méthylbenzylamine.

5) - Composition herbicide caractérisée en ce qu'elle contient comme matière active au moins un composé selon l'une des revendications 1 à 4.

6) - Procédé de désherbage sélectif de cultures notamment le coton et le soja caractérisé en ce qu'on applique sur les plantes et/ou sur le sol de la zone à désherber une quantité efficace d'au moins un composé selon l'une des revendications 1 à 4.

7) - Procédé de préparation d'un composé de formule (III)

$$R - CO - R_5 \qquad (III)$$

dans laquelle $R_5$ représente un reste organique et R représente le radical (II)

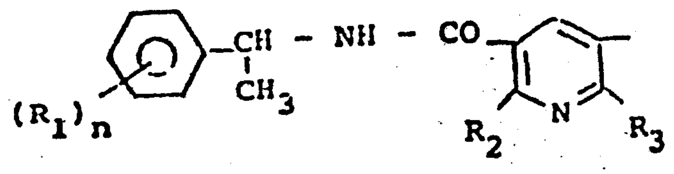

(II)

dans lequel $R_1$, $R_2$, $R_3$ et n ont la même signification que dans la revendication 1, caractérisé en ce qu'il consiste à faire réagir le composé organomagnésien de formule (IV)

$$R_5MgX \qquad (IV)$$

dans laquelle $R_5$ a la même signification que précédemment et X représente un atome d'halogène, sur l'alkoxycarbonylpyridine de formule (V)

$$R - COO - R_6 \qquad (V)$$

dans laquelle R a la même signification que précédemment et $R_6$ représente un radical alkyle comportant de 1 à 6 atomes de carbone.

8) - Procédé de préparation d'un composé de formule (VII)

$$R - CH_2 - N_3 \qquad (VII)$$

dans laquelle R a la même signification que dans la revendication 7, caractérisé en ce qu'il consiste à faire réagir un azidure de métal alcalin sur la chlorométhylpyridine de formule (VIII)

$$R - CH_2 - Cl \qquad (VIII)$$

dans laquelle R a la même signification que précédemment.

9) - Procédé de préparation d'un composé de formule :

$$R - CH_2 - A$$

dans laquelle R a la même signification que dans la revendication 7 et A représente un radical hétérocyclique, caractérisé en ce qu'il consiste à faire réagir la chlorométhylpyridine de formule

$$R - CH_2 - Cl$$

dans laquelle R a la même signification que précédemment, sur le composé hétérocyclique de formule AH, dans laquelle A la même signification que précédemment.

10) - Procédé de préparation d'un composé de formule (X)

$$R - CH_2 - COO - R_6 \qquad (X)$$

dans laquelle R a la même signification que dans la revendication 7 et $R_6$ représente un radical alkyle comportant de 1 à 6 atomes de carbone, caractérisé en ce qu'il consiste à faire réagir un alcanol inférieur sur la cyanométhylpyridine de formule :

$$R - CH_2 - CN \qquad (XI)$$

dans laquelle R a la même signification que précédemment.

11) - Procédé de préparation d'un composé de formule

$$R - R_9$$

dans laquelle R a la même signification que dans la revendication 7 et $R_9$ représente un radical hydroxyalkyle comportant de 2 à 5 atomes de carbone caractérisé en ce qu'il consiste :

- à réduire l'alkoxycarbonylméthylpyridine de formule (X) :

$$R - CH_2 - COO - R_6 \quad (X)$$

dans laquelle R a la même signification que précédemment et $R_6$ représente un radical alkyle $(C_1-C_6)$ pour donner le composé de formule (XII) :

$$R - CH_2 - CH_2 - OH \quad (XII)$$

- ou à réduire l'alcanoylpyridine de formule (XVIII) :

$$R - CO - R_8 \quad (XVIII)$$

dans laquelle R a la même signification que précédemment et $R_8$ représente un radical alkyle comportant de 1 à 4 atomes de carbone, pour donner le composé de formule (IX) :

$$- R - CH (OH) - R_8 \quad (IX)$$

dans laquelle R et $R_8$ ont la même signification que précédemment,

- ou encore à transformer par des méthodes connues les composés de formule (XII) et (IX) en d'autres composés répondant à la formule $R-R_9$ telle que définie ci-dessus.

12) - Procédé de préparation d'un composé de formule (XX) :

$$R - R_{10} \qquad (XX)$$

dans laquelle R a la même signification que dans la revendication 7 et $R_{10}$ représente un radical halogénoalkyle comportant de 2 à 5 atomes de carbone, caractérisé en ce qu'il consiste à halogéner une hydroxyalkylpyridine répondant à la formule :

$$R - R_9$$

dans laquelle R a la même signification que précédemment et $R_9$ a la même signification que dans la revendication 11.

13) - Procédé de préparation d'un composé de formule (XIV)

$$R - (C_pH_{2p+1}) \qquad (XIV)$$

dans laquelle R a la même signification que dans la revendication 7 et p est un entier de 1 à 4, caractérisé en ce qu'il consiste à réduire la chloroalkylpyridine de formule (XVI)

$$R - (C_pH_{2p}) - Cl$$

dans laquelle R et p ont la même signification que précédemment.

14) - Procédé de préparation d'un composé de formule :

$$R - CH = CH_2$$

dans laquelle R a la même signification que dans la revendication 7 caractérisé en ce qu'il consiste à déshydrochlorer la chloroéthylpyridine de formule (XIII)

$$R - CH_2 - CH_2 - Cl \qquad (XIII)$$

dans laquelle R a la même signification que précédemment.

15) - Procédé de préparation d'un composé de formule (XV)

$$R - (C_pH_{2p})-O-R_7 \qquad (XV)$$

dans laquelle R a la même signification que dans la revendication 7, p est égal à 1, 2 ou 3, et $R_7$ représente un radical alcynyle ($C_3$ - $C_7$), caractérisé en ce qu'il consiste à faire réagir l'alcool de formule $R_7$-OH dans laquelle $R_7$ a la même signification que précédemment, sur la chloroalkylpyridine de formule (XVI)

$$R - (C_pH_{2p})- Cl \qquad (XVI)$$

dans laquelle R et p ont la même signification que précédemment.

REVENDICATIONS POUR L'AUTRICHE

1) - Composition herbicide caractérisée en ce qu'elle contient comme matière active au moins un composé selon la formule (I) ci-après :

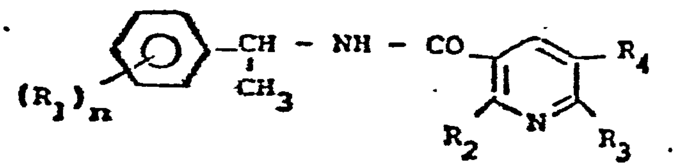
(I)

dans laquelle :

$R_1$ représente un atome d'halogène ou un radical alkyle comportant de 1 à 6 atomes de carbone, éventuellement substitué par un ou plusieurs atomes d'halogène, ou un radical alkoxy comportant de 1 à 4 atomes de carbone,

n est un nombre entier de 0 à 5, valeurs limites comprises étant entendu que lorsque plusieurs substituants $R_1$ sont présents, ces substituants peuvent être soit identiques, soit différents,

$R_2$ et $R_3$, identiques ou différents représentent un radical alkyle comportant de 1 à 3 atomes de carbone ou un radical alkoxy comportant de 1 à 4 atomes de carbone ou un radical alkoxyalkyle comportant de 2 à 8 atomes de carbone,

$R_4$ représente un radical choisi parmi les radicaux acyle ; azidométhyle ; alkoxycarbonylméthyle comportant de 3 à 8 atomes de carbone ; hydroxyalkyle comportant de 2 à 5 atomes de carbone ; halogénoalkyle comportant de 2 à 5 atomes de carbone ; alkyle comportant de 1 à 4 atomes de carbone, méthyle substitué par un radical hétérocyclique comportant de 5 à 6 chaînons et de 1 à 3 hétéroatomes choisis

parmi les atomes d'oxygène, de soufre et d'azote ; vinyle et alcynyloxyalkyle comportant de 4 à 9 atomes de carbone,

ou un sel acceptable en agriculture de ce dérivé de benzylcarbamoylpyridine,

ou une forme isomère, optiquement active, de ce dérivé de la benzylcarbamoylpyridine, seule ou en mélange avec une autre forme isomère optiquement active, de ce dérivé.

2) - Composition selon la revendication 1 caractérisée en ce qu'elle contient comme matière active un composé selon la formule (I), dans laquelle :

n est égal à 0 ou à 1 ou à 2 ou à 3,

$R_1$ représente un atome d'halogène ou le radical méthyle,

$R_2$ représente le radical méthyle,

$R_3$ représente le radical méthyle ou méthoxyméthyle,

$R_4$ représente un radical alcanoyle comportant de 1 à 6 atomes de carbone, azidométhyle, halogénoalkyle comportant de 2 à 5 atomes de carbone, ou alkyle comportant de 1 à 3 atomes de carbone,

ou un sel de ce composé formé avec un acide approprié,

ou une forme isomère optiquement active, seule ou en mélange avec une autre forme isomère optiquement active.

3) -Composition selon la revendication 2 caractérisée en ce qu'elle contient comme matière active un composé selon la formule (I), dans laquelle :

n est égal à zéro, $R_2$ et $R_3$ représentent le radical méthyle, $R_4$ représente un radical alcanoyle comportant de 1 à 3 atomes de carbone, azidométhyle, halogénoalkyle comportant de 2 à 4 atomes de carbone ou alkyle comportant de 1 à 3 atomes de carbone,

ou un sel acceptable en agriculture de ce composé, ou une forme isomère, optiquement active, seule ou en

mélange avec d'autres formes isomères de ce même composé.

4) - Composition selon l'une des revendications 1 à 3 caractérisée en ce que la matière active est un composé de formule (I) sous forme d'isomère optique de même configuration que la (S)-alpha-méthylbenzylamine.

5) - Composition selon l'une des revendications 1 à 4 caractérisée en ce qu'elle contient en poids de 0,05 à 95 % en poids de matière active.

6) - Procédé de désherbage sélectif de cultures, notamment du coton et du soja caractérisé en ce que on applique sur les plantes et/ou sur le sol une quantité efficace d'un composé selon la formule (I) dans laquelle les substituants ont la même signification que dans la revendication 1.

7) - Procédé de préparation d'un composé de formule (III)

$$R - CO - R_5 \qquad (III)$$

dans laquelle $R_5$ représente un reste organique et R représente le radical (II)

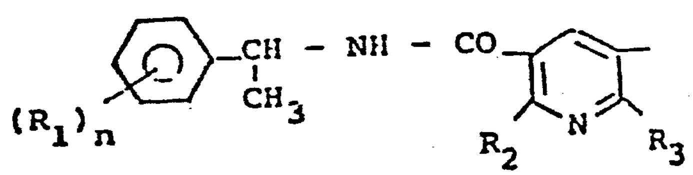

(II)

dans lequel $R_1$, $R_2$, $R_3$ et n ont la même signification que dans la revendication 1, caractérisé en ce qu'il consiste à faire réagir le composé organomagnésien de formule (IV)

$$R_5MgX \qquad (IV)$$

dans laquelle $R_5$ a la même signification que précédemment et X représente un atome d'halogène, sur l'alkoxy-

carbonylpyridine de formule (V)

$$R - COO - R_6 \quad (V)$$

dans laquelle R a la même signification que précédemment et $R_6$ représente un radical alkyle comportant de 1 à 6 atomes de carbone.

8) - Procédé de préparation d'un composé de formule (VII)

$$R - CH_2 - N_3 \quad (VII)$$

dans laquelle R a la même signification que dans la revendication 7, caractérisé en ce qu'il consiste à faire réagir un azidure de métal alcalin sur la chlorométhylpyridine de formule (VIII)

$$R - CH_2 - Cl \quad (VIII)$$

dans laquelle R a la même signification que précédemment.

9) - Procédé de préparation d'un composé de formule :

$$R - CH_2-A$$

dans laquelle R a la même signification que dans la revendication 7 et A représente un radical hétérocyclique, caractérisé en ce qu'il consiste à faire réagir la chlorométhylpyridine de formule

$$R - CH_2-Cl$$

dans laquelle R a la même signification que précédemment, sur le composé hétérocyclique de formule AH, dans laquelle A la même signification que précédemment.

10) - Procédé de préparation d'un composé de formule (X)

$$R - CH_2-COO-R_6 \quad (X)$$

dans laquelle R a la même signification que dans la revendication 7 et $R_6$ représente un radical alkyle comportant de 1 à 6 atomes de carbone, caractérisé en ce qu'il consiste à faire réagir un alcanol inférieur sur la cyanométhylpyridine de formule :

$$R - CH_2 - CN \qquad (XI)$$

dans laquelle R a la même signification que précédemment.

11) - Procédé de préparation d'un composé de formule

$$R - R_9$$

dans laquelle R a la même signification que dans la revendication 7 et $R_9$ représente un radical hydroxyalkyle comportant de 2 à 5 atomes de carbone caractérisé en ce qu'il consiste :

- à réduire l'alkoxycarbonylméthylpyridine de formule (X) :

$$R - CH_2 - COO - R_6 \qquad (X)$$

  dans laquelle R a la même signification que précédemment et $R_6$ représente un radical alkyle ($C_1$-$C_6$) pour donner le composé de formule (XII) :

$$R - CH_2 - CH_2 - OH \qquad (XII)$$

- ou à réduire l'alcanoylpyridine de formule (XVIII) :

$$R - CO - R_8 \qquad (XVIII)$$

  dans laquelle R a la même signification que précédemment et $R_8$ représente un radical alkyle comportant de 1 à 4 atomes de carbone, pour donner le composé de formule (IX) :

$$- R - CH (OH) - R_8 \qquad (IX)$$

dans laquelle R et $R_8$ ont la même signification que précédemment,

- ou encore à transformer par des méthodes connues les composés de formule (XII) et (IX) en d'autres composés répondant à la formule $R-R_9$ telle que définie ci-dessus.

12) - Procédé de préparation d'un composé de formule (XX) :

$$R - R_{10} \qquad (XX)$$

dans laquelle R a la même signification que dans la revendication 7 et $R_{10}$ représente un radical halogénoalkyle comportant de 2 à 5 atomes de carbone, caractérisé en ce qu'il consiste à halogéner une hydroxyalkylpyridine répondant à la formule :

$$R - R_9$$

dans laquelle R a la même signification que précédemment et $R_9$ a la même signification que dans la revendication 11.

13) - Procédé de préparation d'un composé de formule (XIV)

$$R - (C_pH_{2p+1}) \qquad (XIV)$$

dans laquelle R a la même signification que dans la revendication 7 et p est un entier de 1 à 4, caractérisé en ce qu'il consiste à réduire la chloroalkylpyridine de formule (XVI)

$$R - (C_pH_{2p})- Cl$$

dans laquelle R et p ont la même signification que précédemment.

14) - Procédé de préparation d'un composé de formule :

$$R - CH = CH_2$$

dans laquelle R a la même signification que dans la revendication 7 caractérisé en ce qu'il consiste à déshydrochlorer la chloroéthylpyridine de formule (XIII)

$$R - CH_2 - CH_2 - Cl \qquad (XIII)$$

dans laquelle R a la même signification que précédemment.

15) - Procédé de préparation d'un composé de formule (XV)

$$R - (C_pH_{2p})-O-R_7 \qquad (XV)$$

dans laquelle R a la même signification que dans la revendication 7, p est égal à 1, 2 ou 3, et $R_7$ représente un radical alcynyle ($C_3$ - $C_7$), caractérisé en ce qu'il consiste à faire réagir l'alcool de formule $R_7$-OH dans laquelle $R_7$ a la même signification que précédemment, sur la chloroalkylpyridine de formule (XVI)

$$R - (C_pH_{2p})- Cl \qquad (XVI)$$

dans laquelle R et p ont la même signification que précédemment.

0112262

Office européen des brevets

# RAPPORT DE RECHERCHE EUROPEENNE

Numéro de la demande

EP 83 42 0185

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y,D | EP-A-0 044 262 (RHONE-POULENC)<br>* En entier * | 1-3,5, 6 | C 07 D 213/82<br>C 07 D 401/06<br>A 01 N 43/40<br>A 01 N 43/64 |
| | --- | | |
| Y | DE-B-1 116 669 (KRUGMANN & CO.)<br>* En entier * | 1-3 | |
| | --- | | |
| P,Y | EP-A-0 069 033 (RHONE-POULENC)<br>* En entier * | 1-6 | |
| | ----- | | |
| | | | **DOMAINES TECHNIQUES RECHERCHES (Int. Cl. ³)** |
| | | | C 07 D 213/00<br>C 07 D 401/00<br>A 01 N 43/00 |

Le présent rapport de recherche a été établi pour toutes les revendications

| Lieu de la recherche | Date d'achèvement de la recherche | Examinateur |
|---|---|---|
| LA HAYE | 16-03-1984 | NUYTS A.M.K.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82